# EUROPEAN PATENT APPLICATION

(11) **EP 4 249 046 A1**
(43) Date of publication of application: **27.09.2023**
(21) Application number: 22177945.7
(22) Date of filing: 08.06.2022
(51) Int. Cl.: A61N 1/39, A61N 1/375

(54) **A MANUAL EXTERNAL DEFIBRILLATOR**

(30) Priority: 22.03.2022 TR 202204379
(71) Applicant: Aselsan Elektronik Sanayi ve Ticaret Anonim Sirketi, Ankara (TR)
(72) Inventor: Topcuoglu, Oguzhan, Ankara (TR)
(74) Representative: Yamankaradeniz, Kemal

(57) **Abstract**

The present invention relates to a manual external defibrillator (1) that can be used comfortably even by a single person, reduces the risk of being damaged by contact and impacts, and allows users to interact with the interfaces more easily. The manual external defibrillator (1) comprises the following; a body (5) which has a lower wall (2), an upper wall (3) extending in line with the lower wall (2) at a distance from the lower wall (2), and at least one side wall (4) extending between the lower wall (2) and the upper wall (3) so as to connect the lower wall (2) and the upper wall (3) to each other, defines an empty volume between the lower wall (2), upper wall (3) and side wall (4), electronic/mechanical components placed in the defined volume in the body (5), interfaces arranged on the upper wall (3) and/or side wall (4) and allowing the user to interact with at least some of the electronic/mechanical components, at least two shock applicators (8) which has at least one handle (6) and at least one shock application plate (7) arranged at the tip of the handle (6), which is configured to allow the delivery of an electric shock to the patient's body, and at least one connection slot (9) arranged on the side wall (4) to allow external devices such as measuring devices and electronic/mechanical components and/or interfaces to be connected via at least one cable (C). At least one holder (10) is arranged on the upper wall (3) and/or side wall (4) to hold the shock applicator (8) fixed on the body (5) in a way such that the shock application plate (7) does not contact the body (5).

## Description

### Field of the Invention

The present invention relates to a manual external defibrillator that can be used comfortably even by a single person, reduces the risk of being damaged by contact and impacts, and allows users to interact with the interfaces more easily.

### State of the Art

A defibrillator is a medical device designed to give an electric shock to the patient in cases of health problems such as sudden cardiac arrest or cardiac arrhythmia. Today, the use of devices called manual external defibrillators, which are portable in a case such as a bag, is seen in the state of the art so as to allow emergency response to be performed by people who have received first aid training, without the need to go to the hospital. Manual external defibrillators can be used with the assistance of a plurality of persons or by a single person alone, depending on the situation.

Shock applicators get help from the people next to them to apply gel to the relevant surface of the shock application plate, preferably made of metal, which contacts the patient's body in the state of the art, if the person who takes two external shock applicators to administer therapeutic shock is not alone while using the device If the person is alone at this time, he/she has difficulty in applying gel to the shockers while holding the shockers with both hands because he/she has no free hand. On the other hand, the shock applicator causes the contamination of the chambers when the shock applicator, whose shock application plate is gelled, is placed in the concave-shaped chambers on the device in its gelled form after application to the patient's body. The gels that stick to the chambers and dry out give damage to the mechanical structure inside the chambers and make it difficult to clean the same afterwards.

In addition, manual external defibrillators in the state of the art, the socket-like connection sockets, which enable the defibrillator to be connected to external devices such as measuring devices, preferably via cables, are usually arranged adjacent with the walls on the side or front of the defibrillator body. Users who usually interact with the front and top surfaces of the defibrillator during the usage cycle of the devices, may cause the parts of the connection sockets to be warped, bent and damaged by hitting other objects due to the urgent use and fussy nature of the event, while carrying the defibrillator or moving around the defibrillator. On the other hand, a liquid flowing over the defibrillator may circulate from the side and come to the part where the connection sockets are located, creating a risk of damaging these sockets.

In addition to all of these, users try to fix the overhanging cables by wrapping them around the defibrillator body due to the length of said cables in manual external defibrillators in the state of the art. This situation makes the interaction in this area difficult as the cables pass in front of the screen and keys on the front of the defibrillator. Cables that are not wrapped around the body sometimes creep in places with a worse result; they get entangled with each other and lose their hygienic property.

In another patent document included in the state of the art which is the United States patent document numbered US20180036545 discloses a defibrillator system comprising a computer, a defibrillator element and software configured to transmit data from the defibrillator element to the computer. When a release button or latch release located on the outer surface of said defibrillator device is pressed, a cover protecting the flat or coiled USB cable and plug and another cover protecting the electrode tips and pads are opened.

Therefore, a manual external defibrillator that can be used comfortably even by a single person, reduces the risk of being damaged by contact and impacts, and allows users to interact with the interfaces more easily is required in the state of the art.

### Brief Description of the Invention

The aim of the present invention is to reveal a manual external defibrillator that can be used comfortably even by a single person, reduces the risk of being damaged by contact and impacts, and allows users to interact with the interfaces more easily. The manual external defibrillator as defined in the first claim and the dependent claims so as to achieve the aim of the present invention comprising of the following; a body having a lower wall, an upper wall extending in line with the lower wall at a distance from the lower wall, and at least one side wall extending between the lower wall and the upper wall in such a way as to connect the lower wall and the upper wall so as to define an empty volume between them, electronic/mechanical components placed in the defined volume in the body and enabling the defibrillator to operate and be controlled, interfaces arranged on the upper wall and/or side wall and allowing the user to interact with at least some of the electronic/mechanical components to control and/or monitor the operation of the electronic/mechanical components, at least two shock applicators having a handle that can be grasped by the user, and at least one shock delivery plate arranged at the tip of the handle, which is configured to allow the delivery of an electric shock to the patient's body when it comes into contact with the patient's body, and at least one connection slot arranged on the sidewall to allow external devices such as measuring devices and electronic/mechanical components and/or interfaces to be connected via at least one cable, manual external defibrillator further comprises at least one holder arranged on the upper wall and/or side wall to hold the shock applicator still in a way such that the shock delivery plate does not contact the body. It is ensured that a single first aider can gel the relevant surfaces of the shock applicators in a short time without any difficulty with the help of the holder in the inventive manual external defibrillator, the body is prevented from getting dirty by dripping gel on the body during this gelling process.

### Detailed Description of the Invention

Manuel external defibrillator realized to achieve the aim of the present invention is shown in the attached figures, in which;
Figure - 1 is a perspective view of an embodiment of the inventive manual external defibrillator.
Figure - 2 is another perspective view of an embodiment of the inventive manual external defibrillator.
Figure - 3 is another perspective view of an embodiment of the inventive manual external defibrillator.
Figure - 4 is another perspective view of an embodiment of the inventive manual external defibrillator.

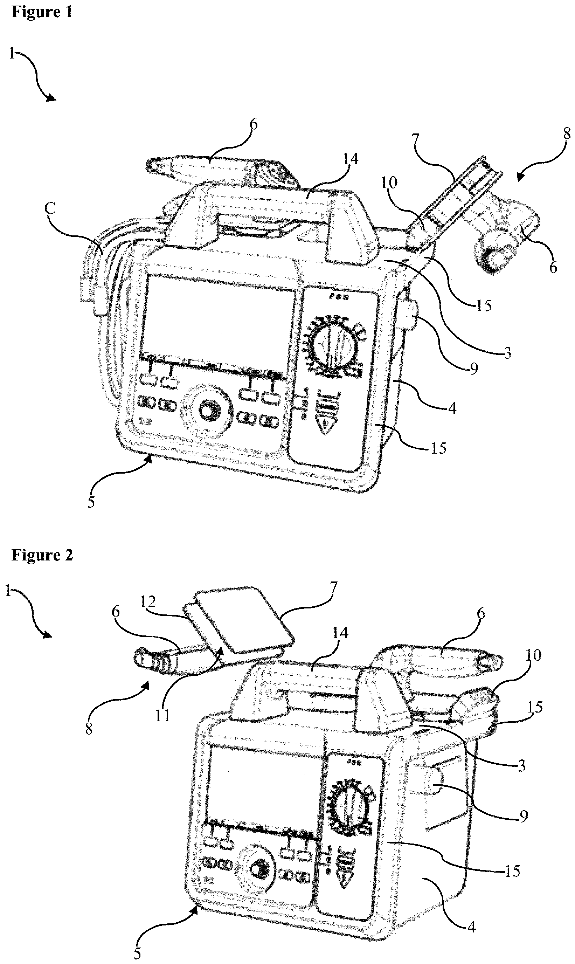

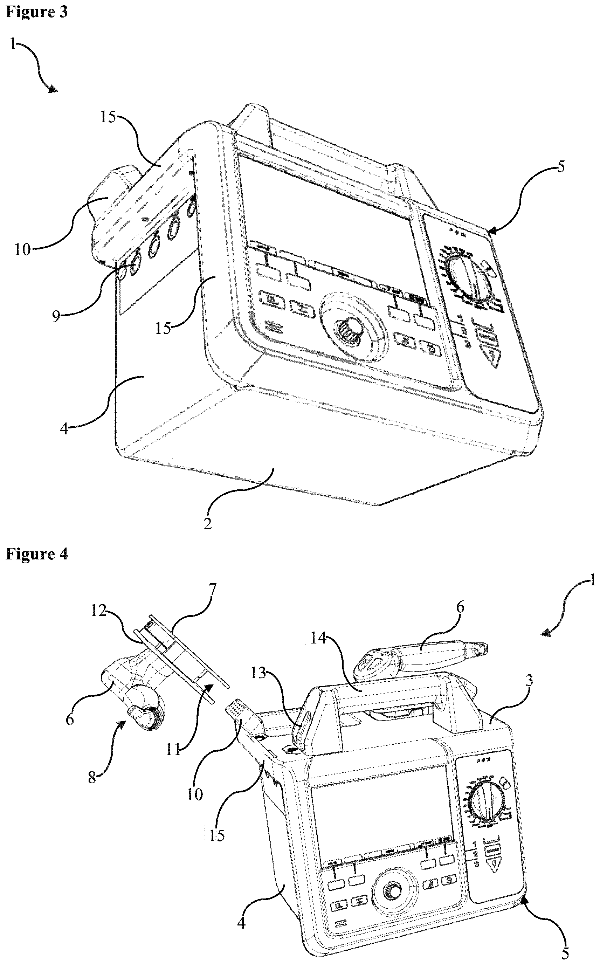

The parts in the figure are enumerated one by one and the parts correspond to these numbers are given in the following.
1. Manual external defibrillator
2. Lower wall
3. Upper wall
4. Side wall
5. Body
6. Handle
7. Shock application plate
8. Shock applicator
9. Connection socket
10. Holder
11. Gap
12. Support plate
13. Hanger
14. Handgrip
15. Extension
C. Cable

The manual external defibrillator (1) comprises the following; a body (5) which has a lower wall (2), an upper wall (3) extending in line with the lower wall (2) at a distance from the lower wall (2), and at least one side wall (4) extending between the lower wall (2) and the upper wall (3) so as to connect the lower wall (2) and the upper wall (3) to each other, defines an empty volume between the lower wall (2), upper wall (3) and side wall (4), electronic/mechanical components (not shown in the figures) placed in the defined volume in the body (5), interfaces (not shown in the figures) arranged on the upper wall (3) and/or side wall (4) and allowing the user to interact with at least some of the electronic/mechanical components, at least two shock applicators (8) which has at least one handle (6) and at least one shockapplication plate (7) arranged at the tip of the handle (6), which is configured to allow the delivery of an electric shock to the patient's body, and at least one connection slot (9) arranged on the side wall (4) to allow external devices such as measuring devices and electronic/mechanical components and/or interfaces to be connected via at least one cable (C). The manual external defibrillator (1) body (5) preferably has a hollow prismatic shape and comprises the lower wall (2), the upper wall (3) and at least one side wall (4) surrounding the empty space therein. Electronic and mechanical components that enable the manual external defibrillator (1) to operate, such as the power supply, electronic circuit boards, fixing elements, are kept in the empty space within the body (5). Interfaces such as buttons, keys, and screens that allow interaction with electronic and mechanical components are preferably arranged on the side wall (4) so as to control the operation of said electronic and mechanical components and to observe their operating conditions. The shock applicator (8) contains a handle (6) that the user can easily grasp and hold in his hand, and at least one shock application plate (7) arranged at one end of the handle (6), which allows electric shock to be applied to the patient's body by contacting the patient's body. The shock applicaiton plate (7) is preferably a planar extending metallic plate. Connection socket (9) arranged on at least one of the side walls (4) of the body (5) enables external devices such as measuring devices to be communicated with the electronic components inside the body (5) or with the interfaces arranged on the side wall (4) by means of cables (C) to provide data communication. Said connection sockets (9) can be formed, for example, in the form of an open hole on the side wall (4), or in the form of a socket extending from the side wall (4) to the outside of the body (5). When an electric shock is required to be given to a patient in an emergency, the patient-contacting surface of the shock applicaiton plate (7) is gelled by grasping the shock applicator (8) by its handle (6) immediately after the electronic/mechanical components are powered on or through the interfaces and then the patient's body is brought to the position to be contacted, and electric shock is applied to the patient's body. The intensity of said electric shock can be adjusted by controlling electronic/mechanical components via interfaces.

The inventive manual external defibrillator (1) comprises at least one holder (10) arranged on the upper wall (3) and/or the side wall (4) to keep the shock applicator (8) fixed on the body (5) so that the shock application plate (7) does not contact the body (5). In the preferred embodiment, the holder (10) is arranged on the upper wall (3) of the body (5) in the most appropriate place that provides ease of access; this situation provides easy access to the first aider during the emergency response, thus minimizing the loss of time during the first aid. Since the first aider has to gel the shock application plate (7) before starting the first aid using the shock applicator (8), when not in use, he/she takes the shock applicator (8), which is preferably kept in a cavity arranged on the upper wall (3), out of the cavity by grasping its handle (6) and gels the surface of the shock application plate (7) that will contact the patient's body and attaches the relevant shock applicator (8) to the holder (10). The gelled surface of the shock applicator (8) is kept fixed on the body (5) without contacting the body (5) by connecting the shock applicator (8) with the holder (10) in this manner. The first aider then gels the other shock applicator (8) in the manual external defibrillator (1) in the same way and the previously gelled shock applicator (8) held by the holder (10) is also grasped by its handle (6) and separates it from the holder (10). After the first aider grasps both shock applicators (8) by the handles (6), he/she performs the first aid operation by bringing the shock application plate (7) of both shock applicators (8) into contact with the patient's body and giving the body an electric shock. Thus, for proper first aid, the risk of mechanical failure that may occur over time due to the gel accumulation on the body (5) is eliminated by preventing the body (5) from being contaminated by the gel during the gelling of the second shock applicator (8), when the first shock applicator (8) is held by the holder (10) in such a way that the gels do not spill onto the body (5).

In one embodiment of the present invention, the shock applicator (8) includes a support plate (12) that extends parallel to the shock application plate (7) so that there is a gap (11) between the shock application plate (7) on the handle (6), in the embodiment the holder (10) is arranged as a protrusion having the appropriate thickness to enter into the gap (11) located between the shock application plate (7) and the support plate (12), extending on the upper wall (3) and/or the side wall (4) at an angle with respect to the extension axis of the upper wall (3) and/or the side wall (4). In a preferred embodiment, the holder (10) in the form of protrusion is arranged to make an angle of almost 40 degrees with the extension axis of the upper wall (3) and/or the side wall (4). The holder (10) that has the form of a protrusion with a thickness that allows it to enter the gap (11) located on the shock applicator (8) provides both ease of production and therefore cost advantage and also ensures that the loss of time is minimized by the fact that the shock applicator (8) can be placed on the protrusion quite easily by means of the gap (11).

In an embodiment of the invention, the manual external defibrillator (1) also has at least one hanger (13) arranged on the upper wall (3) and/or at least one side wall (4) and adapted to have a structure that allows the cables (C) to be wound on it In a preferred embodiment of the present invention, said hanger (13) is arranged on the upper wall (3) in the most appropriate place that provides ease of access, this situation provides easy access to the first aider during the emergency response, thus minimizing the loss of time during the first aid. It is ensured that said cables (C) are collected in a certain place, thus preventing the cable (C) clutter by hanging the cables (C) connecting the electronic components inside the body (5) or the interfaces on the side wall (4) with external devices, on a hanger (13). The loss of time due to accessing the interfaces is eliminated during the use of the manual external defibrillator (1) By preventing cable (C) clutter, preventing that the cables (C) prevent access to the interfaces arranged on the side wall (4).

In one embodiment of the present invention, the hanger (13) is adapted as a protrusion on the upper wall (3) arranged at an angle to the axis on which the upper wall (3) extends. The hanger (13) which has the form of a protrusion arranged on the upper wall (3) provides both ease of production and therefore cost advantage, also ensures that the cables (C) are easily wrapped around the protrusion with easy access, thus minimizing the loss of time.

In an embodiment of the invention, the manual external defibrillator (1) also includes at least one handgrip (14) arranged on the body (5), in this embodiment, the hanger (13) is integrated on the handgrip (14). In said application, space is saved on the body (5) with the help of the integration of the hanger (13) on the handgrip (14), also production and cost advantage is provided by producing the handgrip (14) and the hanger (13) as one piece.

In an embodiment of the invention, the manual external defibrillator (1) comprises extensions (15) that are arranged on the side adjacent to at least one side wall (4) having a connection socket (9) on the upper wall (3) and at least one side wall (4) and extending outward from the body (5). In a preferred embodiment, said extensions (15) have a length that allows moving away from the body (5) by almost 20 millimeters. The connection sockets (9) are surrounded by said extensions (15), so that they are protected on the side wall (4). In this way, the connection sockets (9) arranged on the related side wall (4) are protected from the liquids flowing from the upper wall (3) and the dangers arising from external impacts, thus their long service life is ensured.

It is ensured that a single first aider can gel the relevant surfaces of the shock applicators (8) in a short time without any difficulty with the help of the holder (10) in the inventive manual external defibrillator (1), the body (5) is prevented from getting dirty by dripping gel on the body (5) during this gelling process. The cables (C) that enable the external devices to be connected to the electronic components or interfaces that enable the operation and control of the manual external defibrillator (1) are kept in a collective way and the cable (C) clutter is eliminated with the help of the hanger (13). The connection sockets (9) arranged on the related side wall (15) are surrounded and protected from external dangers with the help of the extensions (15) extending from the upper wall (3) or at least one of the side walls (4) to the outside of the body (5).

It is possible to develop a variety of applications of the inventive manual external defibrillator (1) , the invention is not limited to the examples described herein, it is essentially as claimed in the claims.

## Claims

1. A manual external defibrillator (1) **comprises of**; a body (5) which has a lower wall (2), an upper wall (3) extending in line with the lower wall (2) at a distance from the lower wall (2), and at least one side wall (4) extending between the lower wall (2) and the upper wall (3) so as to connect the lower wall (2) and the upper wall (3) to each other, defines an empty volume between the lower wall (2), upper wall (3) and side wall (4), electronic/mechanical components placed in the defined volume in the body (5), arranged on the upper wall (3) and/or side wall (4) and allowing the user to interact with at least some of the electronic/mechanical components, at least two shock applicators (8) which has at least one handle (6) and at least one shock application plate (7) arranged at the tip of the handle (6), which is configured to allow the delivery of an electric shock to the patient's body, and at least one connection socket (9) arranged on the side wall (4) to allow external devices such as measuring devices and electronic/mechanical components and/or interfaces to be connected via at least one cable (C) and **characterized by comprising;** at least one holder (10) arranged on the upper wall (3) and/or side wall (4) to hold the shock applicator (8) fixed on the body (5) in a way such that the shock application plate (7) is does not contact the body (5).

2. Manual external defibrillator (1) according to claim 1, **characterized by comprising;** a shock applicator (8) that includes a support plate (12) that extends parallel to the shock application plate (7) so that there is a gap (11) between the shock application plate (7) on the handle (6), a holder (10) that is arranged as a protrusion having the appropriate thickness to enter into the gap (11) located between the shock application plate (7) and the support plate (12), extending on the upper wall (3) and/or the side wall (4) at an angle with respect to the extension axis of the upper wall (3) and/or the side wall (4).

3. Manual external defibrillator (1) according to claim 1 or 2, **characterized by comprising;** at least one hanger (13) that is arranged on the upper wall (3) and/or at least one side wall (4) and adapted to have a structure that allows the cables (C) to be wound thereon.

4. Manual external defibrillator (1) according to claim 3, **characterized by comprising;** the hanger (13) that is adapted as a protrusion on the upper wall (3) arranged at an angle to the axis on which the upper wall (3) extends.

5. Manual external defibrillator (1) according to claim 3 or 4, **characterized by comprising;** at least one handgrip (14) arranged on the body (5) and a hanger (13) that is integrated on the handgrip (14).

6. Manual external defibrillator (1) according to any of the preceding claims, **characterized by comprising;** extensions (15) that are arranged on the side adjacent to at least one side wall (4) having a connection socket (9) on the upper wall (3) and at least one side wall (4) in a way extending outward from the body (5).
